(19) Europäisches Patentamt / European Patent Office / Office européen des brevets

(11) **EP 4 230 227 A1**

(12) **EUROPEAN PATENT APPLICATION**
published in accordance with Art. 153(4) EPC

(43) Date of publication:
**23.08.2023 Bulletin 2023/34**

(21) Application number: **21886309.0**

(22) Date of filing: **28.10.2021**

(51) International Patent Classification (IPC):
**A61K 49/06** (2006.01)  **A61B 5/055** (2006.01)

(52) Cooperative Patent Classification (CPC):
**A61B 5/055; A61K 49/06; A61P 19/02**

(86) International application number:
**PCT/JP2021/039798**

(87) International publication number:
**WO 2022/092192 (05.05.2022 Gazette 2022/18)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **28.10.2020 JP 2020180918**

(71) Applicants:
• **National University Corporation
  Hokkaido University
  Sapporo-shi, Hokkaido 060-0808 (JP)**
• **Taiyo Nippon Sanso Corporation
  Tokyo 142-8558 (JP)**

(72) Inventors:
• **ONODERA, Tomohiro
  Sapporo-shi, Hokkaido 060-0808 (JP)**
• **KUDO, Kohsuke
  Sapporo-shi, Hokkaido 060-0808 (JP)**
• **HOSOKAWA, Yoshiaki
  Sapporo-shi, Hokkaido 060-0808 (JP)**
• **IWASAKI, Norimasa
  Sapporo-shi, Hokkaido 060-0808 (JP)**
• **KAMEDA, Hiroyuki
  Sapporo-shi, Hokkaido 060-0808 (JP)**

(74) Representative: **Prüfer & Partner mbB
Patentanwälte · Rechtsanwälte
Sohnckestraße 12
81479 München (DE)**

(54) **CONTRAST AGENT FOR DETECTING CARTILAGE DAMAGE, AND METHOD AND PROGRAM FOR DETECTING CARTILAGE DAMAGE USING SAID CONTRAST AGENT**

(57)   The present invention provides: an MRI contrast agent for detecting cartilage damage, which comprises $^{17}O$-labeled water; and a method and a program for detecting cartilage damage using the contrast agent. According to the present invention, it becomes possible to detect a damage in a cartilage, particularly a minor damage in the surface layer of a cartilage which has been difficult to detect so far, by using $^{17}O$-labeled water as a contrast agent.

Fig.3

A.

EP 4 230 227 A1

**Description**

Field

The present invention relates to a contrast agent for detecting cartilage damage, containing $^{17}$O-labeled water and a method and a program for examining cartilage damage using the contrast agent.

Background

Osteoarthritis is a disease causing an inflammation localized within joints accompanied by degeneration and fracture of articular cartilage, proliferative changes in joint margin and subchondral bone, and synovitis. In patients with osteoarthritis, there is a problem in that pain interferes with daily life activities including walking, resulting in reduced quality of life (QOL).

Articular cartilage contains water, which makes up 70% of its components, and this water is held by proteoglycans, in about 10%, present in intercellular spaces. Osteoarthritis, which progresses following overloads or injuries during exercise, is thought to be caused by disruption of the hydrostatic pressure maintenance mechanism of cartilage tissue in an early stage of damage, leading to induction of chondrocyte apoptosis. Thus, a method of diagnosis focusing on the water-retaining function of cartilage is important.

In an early image diagnosis of articular cartilage, various imaging sequences of magnetic resonance imaging (MRI) are often used. T2 mapping is a method of imaging capable of evaluating irregular collagen arrangement and increased water content. In making a diagnosis based on T2 mapping, evaluation is required to be performed with consideration on a difference in T2 values due to the different arrangement directions of collagen between the surface layer, the intermediate layer, and the deep layer of the cartilage surface. In addition, caution is also required for the possibility of normal cartilage being interpreted as degenerated cartilage due to elongation of the T2 value by what is called magic angle effect. T1$\rho$ mapping is capable of detecting a proteoglycan concentration, which reduces along with cartilage degeneration, and is regarded as being less affected by the magic angle effect, but it is required to be taken into account that T1$\rho$ mapping is also affected by a water content. Delayed gadolinium enhanced magnetic resonance imaging of cartilage (dGEMRIC), which is a contrast imaging examination, is a method of imaging using gadolinium and has high specificity to a proteoglycan concentration change in cartilage, but a gadolinium contrast agent is required to be administered in an amount double a normal administration amount, causing an invasiveness problem.

None of the above methods has enough sensitivity and specificity to evaluate metabolic dynamic changes in cartilage matrix, and no appropriate modalities for diagnosing early osteoarthritis have been developed.

Water labeled with oxygen-17 ($^{17}$O), which is a stable isotope of oxygen, (also referred as oxygen-17 stable isotope-labeled water, $H_2$-$^{17}$O-containing water, and $^{17}$O-labeled water, and denoted as $^{17}$O-labeled water in the present specification) is known as an effective component of a nuclear magnetic resonance image diagnostic agent (Patent Literature 1), and methods of MRI imaging for evaluating a cerebral blood flow disorder using $^{17}$O-labeled water are being developed (Patent Literature 2 and Non Patent Literature 1). Owing to its safety, $^{17}$O-labeled water is expected to provide further clinical applications.

Citation List

Non Patent Literature

Patent Literature 1: JP 2003-102698 A
Patent Literature 2: JP 2013-255586 A

Patent Literature

Non Patent Literature 1: Kudo K. et al., Magn Reson Med Sci 2018 17(3): 223-230.

Summary

Technical Problem

An object of the present invention is to provide new means useful for detecting cartilage damage and performing an image diagnosis of early osteoarthritis.

Solution to Problem

The inventors of the present invention have found that administration of $^{17}O$-labeled water promptly increases the $^{17}O$ concentration in a damaged cartilage region, but such an increase in the $^{17}O$ concentration does not occur in a normal cartilage region to complete the following invention.

Item 1. An MRI contrast agent for detecting cartilage damage, the MRI contrast agent including $^{17}O$-labeled water.

Item 2. A method for examining cartilage damage, the method including:

acquiring a signal value derived from $^{17}O$ from MRI images of cartilage of a subject to which an MRI contrast agent including $^{17}O$-labeled water has been administered before and after administration of the contrast agent; comparing the signal values or $^{17}O$ concentrations calculated from the signal values between before and after administration of the contrast agent; and determining a change in the signal value or an increase in the $^{17}O$ concentration with administration of the contrast agent.

Item 3. The method according to Item 2, wherein the cartilage is articular cartilage.

Item 4. The method according to Item 2 or 3, wherein the contrast agent is administered into a joint cavity of the subject.

Item 5. The method according to any one of Items 2 to 4, wherein the MRI images are T2-weighted images taken with a proton MRI apparatus, and the change in the signal value is a reduction in the signal value.

Item 6. A program for examining cartilage damage for executing processing to acquire a signal value derived from $^{17}O$ from MRI images of cartilage of a subject to which an MRI contrast agent including $^{17}O$-labeled water has been administered before and after administration of the contrast agent, processing to optionally calculate a $^{17}O$ concentration from the signal value, and processing to compare the signal values or the $^{17}O$ concentrations calculated from the signal values between before and after administration of the contrast agent.

Item 7. The program according to Item 6, wherein the cartilage is articular cartilage. Item 8. The program according to Item 6 or 7, wherein the contrast agent is administered into a joint cavity of the subject.

Item 9. The program according to any one of Items 6 to 8, wherein the MRI images are T2-weighted images taken with a proton MRI apparatus, and the change in the signal value is a reduction in the signal value.

Advantageous Effects of Invention

According to the present invention, by using $^{17}O$-labeled water as a contrast agent, a highly safe method of image diagnosis that can detect cartilage damage, especially slight damage to the cartilage surface layer, which is an early lesion of osteoarthritis, which has been difficult to detect.

Brief Description of Drawings

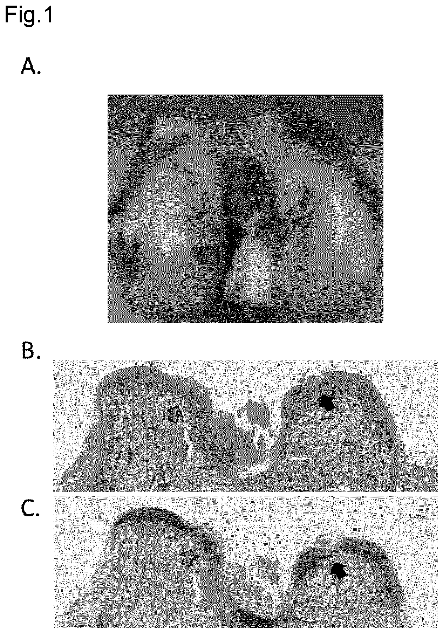

FIG. 1A is an appearance photograph of rabbit both femoral condyles 4 weeks after anterior cruciate ligament transection stained with India ink, FIG. 1B is an HE-stained image of a sliced specimen of both femoral condyles, and FIG. 1C is a Safranin-O-stained image of the sliced specimen of both femoral condyles.

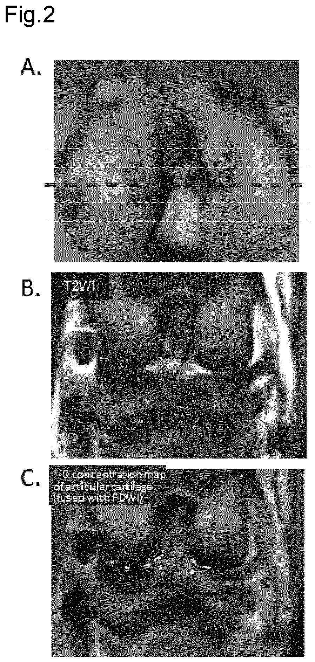

FIG. 2A is a diagram in which the positions of MRI imaging sections are indicated by five dotted lines on the India ink-stained image of the rabbit both femoral condyles 4 weeks after anterior cruciate ligament transection. FIG. 2B is a T2-weighted image (before administration of $^{17}O$-labeled water) on the imaging section corresponding to the central dotted line out of the five dotted lines in FIG. 2A, and FIG. 2C is a diagram in which a $^{17}O$ concentration map of articular cartilage based on the T2-weighted image is fused with a proton density-weighted image (an anatomical image).

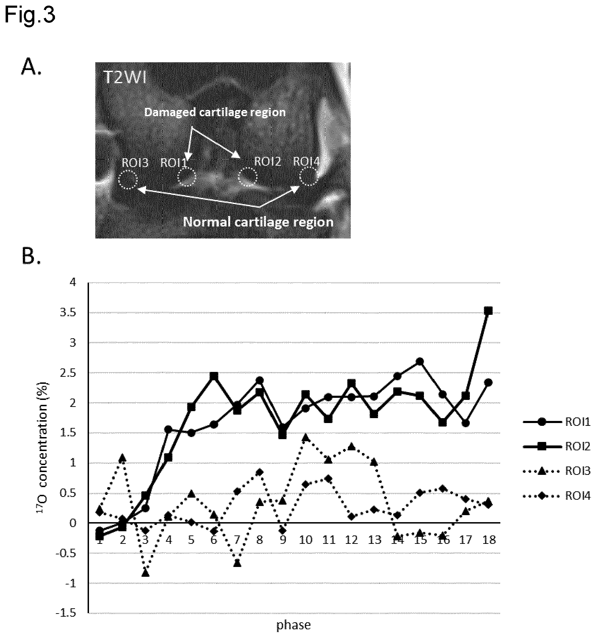

FIG. 3A is a diagram indicating the positions of regions of interest (ROIs) analyzed in the T2-weighted image, and FIG. 3B is a graph indicating changes over time in the $^{17}O$ concentration in the respective ROIs. The phase on the horizontal axis corresponds to the number of times of imaging of MRI. The scan time is 3 minutes 39 seconds.

Description of Embodiments

The description of the present invention described below may be based on representative embodiments or specific examples, but the present invention is not limited to such embodiments or specific examples. In the present specification, the upper limit value and the lower limit value of each numerical value range can be combined as desired. In the present specification, a numerical range represented using "to" or "-" means a range including numerical values at both ends thereof as an upper limit value and a lower limit value, unless otherwise noted.

The present invention provides an MRI contrast agent for detecting cartilage damage, the MRI contrast agent including $^{17}O$-labeled water.

$^{17}O$-labeled water is water containing a higher concentration of $H_2$-$^{17}O$ than naturally occurring water does. It suffices if the concentration of $H_2$-$^{17}O$ in $^{17}O$-labeled water is a concentration that can provide a sufficient concentration of $H_2$-$^{17}O$ for detection by MRI at a site in need of a cartilage damage examination, but a higher concentration is preferred in order to reduce the volume of liquid to be administered to a subject. The $H_2$-$^{17}O$ concentration in $^{17}O$-labeled water is, for example, 5 mol% or more, preferably 10 mol% or more, and more preferably 15 mol% or more. There is no limit to the upper limit of the $H_2$-$^{17}O$ concentration in $^{17}O$-labeled water, which can theoretically be 100 mol%. The mol% representing the $H_2$-$^{17}O$ concentration in $^{17}O$-labeled water is synonymous with atom% (the ratio of oxygen atoms).

$^{17}O$-labeled water can be produced by methods known to those skilled in the art, such as the method described in Japanese Patent Application Laid-open No. 2000-218134, for example, in which $^{17}O$ is concentrated in advance by low-temperature distillation of raw oxygen containing $^{17}O$ and then hydrogen is added to the concentrate to cause them to react with each other. For $^{17}O$-labeled water, commercially available products, such as those sold by Taiyo Nippon Sanso Corporation, can be used.

In addition to $^{17}O$-labeled water, the contrast agent can contain components such as isotonic agents such as sodium chloride, potassium chloride, glucose, D-sorbitol, and glycerin; preservatives such as benzyl alcohol, para-hydroxybenzoate, and chlorobutanol; antioxidants such as ascorbic acid, $\alpha$-tocopherol, and sulfites; and buffers such as phosphates, carbonates, acetates, and citrates. The contrast agent containing such other components as exemplified above in addition to $^{17}O$-labeled water can be represented as a contrast composition.

The contrast agent can be administered by any route of administration to a subject in need of a cartilage damage examination so long as the administered contrast agent is accessible to the cartilage in the subject's body, but local administration near the cartilage is preferred in order to perform the examination with a smaller amount of the contrast agent. When the cartilage to be examined is articular cartilage, the contrast agent is particularly preferably administered locally into a joint cavity.

It suffices if the contrast agent is administered in a dose giving an $H_2$-$^{17}O$ concentration around the cartilage, or an $H_2$-$^{17}O$ concentration in the joint cavity in the case of the local administration to the joint cavity, for example, of 0.5 mol% or more and preferably 0.8 mol% or more, or an amount giving around 1 mol%, for example. The dose can be set as appropriate by those skilled in the art by considering factors such as an $H_2$-$^{17}O$ concentration in the contrast agent, the volume of the joint cavity, and the route of administration.

For the cartilage damage examination, the subject is subjected to MRI imaging of the cartilage before and after administration of the contrast agent, and MRI images are acquired. For the MRI imaging of cartilage, a direct method using the nuclear magnetic resonance phenomenon of $^{17}O$ itself may be used, or an indirect method using a T2 shortening occurring in protons in $H_2$-$^{17}O$ molecules may be used. The indirect method uses the nuclear magnetic resonance phenomenon of protons, thus allowing use of a proton MRI apparatus in current clinical use, and also allowing detection of $^{17}O$ with higher sensitivity than that of the direct method. The following further describes a method of $^{17}O$ detection with the indirect method as an example.

In the indirect method, to detect the T2 shortening with high sensitivity, T2-weighted images (T2WI) are preferably acquired by employing a T2-weighted sequence by the fast spin echo method or the spin echo method, especially the fast spin echo method. Imaging parameters such as a time of repetition (TR) and a time of echo (TE) may be set as appropriate within ranges generally used to acquire T2-weighted images in accordance with an imaging sequence. In the case of the fast spin echo method, a favorable image can be acquired with one phase of 3 minutes 39 seconds with a TR of 1,600 ms, a TE of 129 ms, an FA of 150°, an echo train (ETL) of 12, and a number of times of addition of six times, for example.

Imaging (scanning) is performed one or a plurality of times each before and after administration of the contrast agent. Imaging before administration of the contrast agent may be performed at any point in time before administration, and imaging after administration of the contrast agent may be performed at any point in time from immediately after administration to 3 days after administration. In a preferred embodiment, imaging before administration of the contrast agent is performed at any point in time from 30 minutes before administration to immediately before administration, and imaging after administration of the contrast agent is performed at any point in time from immediately after administration to 60 minutes after administration. In a more preferred embodiment, imaging before administration of the contrast agent is performed immediately before administration, and imaging after administration of the contrast agent is performed continuously from immediately after administration to 10 minutes after administration.

Next, signal values (signal intensity) are acquired from the acquired MRI images by image analysis, and the signal values are compared between before and after administration of the contrast agent.

In one embodiment, any region of interest (ROI) is set in a region corresponding to the cartilage in the MRI images, and signal values are acquired from respective pixels included in the ROI. Identification of the cartilage in the MRI images may be made with reference to other images such as proton density-weighted images. Comparison of the signal values between before and after administration of the contrast agent can be made using a value obtained by averaging the signal values of the respective pixels included in the ROI.

In the indirect method, the T2 shortening occurs in protons in $H_2$-$^{17}O$ molecules, and thus the signal values reduce. Thus, in an ROI in which the average of the signal values has reduced after administration compared to that before administration of the contrast agent, the $H_2$-$^{17}O$ concentration has increased with the administration, and the cartilage contained in the ROI can be evaluated as having damage or being likely to have damage.

In another embodiment, signal values are acquired from respective pixels in the MRI image, and the differences in the signal values between before and after administration of the contrast agent are displayed on a map. In a region in which the signal value has decreased with administration of the contrast agent displayed on the map, the $H_2$-$^{17}O$ concentration has increased with the administration, and the cartilage contained in that region can be evaluated as having damage or being likely to have damage.

Instead of comparing the signal values between before and after administration of the contrast agent as described above, a $^{17}O$ concentration calculated from the signal values may be compared. The $^{17}O$ concentration can be calculated based on the relation between the signal values and the $^{17}O$ concentration obtained in advance. When the signal value is a signal value of the T2-weighted image, for example, the $^{17}O$ concentration can be calculated using the following equation.

[Mathematical 1]

$$^{17}O \text{ concentration (mol\%)} = -1/(TE \times R_2{}^{17}O) \times \log(S/S0) + 0.038$$

In the equation, TE is the time of echo, $R_2{}^{17}O$ is the transverse relaxation capacity of protons in $H_2$-$^{17}O$ molecules (3.33 mol%$^{-1}$s$^{-1}$), S is a signal value acquired from the T2-weighted image after administration of the contrast agent, and S0 is a baseline signal value acquired from the T2-weighted image before administration of the contrast agent. The value 0.038 is the natural abundance of $^{17}O$.

In one embodiment, for example, an average $^{17}O$ concentration in the ROI is calculated from the average of the signal values of the respective pixels included in the ROI, and the average $^{17}O$ concentrations are compared between before and after administration of the contrast agent. When the $^{17}O$ concentration in the ROI increases after administration, the cartilage contained in the ROI can be evaluated as having damage or being likely to have damage.

In another embodiment, $^{17}O$ concentrations are calculated from the signal values of the respective pixels in the MRI image, and the differences in the $^{17}O$ concentrations between before and after administration of the contrast agent are displayed on a map. The cartilage contained in a region with an increased $^{17}O$ concentration with administration of the contrast agent displayed on the map can be evaluated as having damage or being likely to have damage.

When setting ROIs in the MRI image, in addition to an ROI specifying a region to be examined (a test ROI), an ROI as a control (a control ROI) may be set in a normal cartilage part. The averages of the signal values or the $^{17}O$ concentrations in each of the test ROI and the control ROI are compared between before and after administration of the contrast agent, and if the degree of a reduction in the signal values or the degree of an increase in the $^{17}O$ concentration with administration is greater in the test ROI than in the control ROI, or if the rate of reduction in the signal values or rate of increase in the $^{17}O$ concentration with administration is greater in the test ROI than in the control ROI, the cartilage contained in the test ROI can be evaluated as having damage or being likely to have damage.

The above describes MRI imaging using the indirect method as an example. Also when using MRI imaging using the direct method, in which $^{17}O$ itself is to be detected, cartilage damage can be examined in a similar way by acquiring signal values derived from $^{17}O$ from MRI images and based on a change in the signal values or an increase in the $^{17}O$ concentration calculated from the signal values. In this context, the change in the signal values is an increase in the signal values.

Thus, the present invention provides a method for examining cartilage damage, the method including acquiring a signal value derived from $^{17}O$ from MRI images of cartilage of a subject to which an MRI contrast agent including $^{17}O$-labeled water has been administered before and after administration of the contrast agent, comparing the signal values or $^{17}O$ concentrations calculated from the signal values between before and after administration of the contrast agent, and determining a change in the signal value or an increase in the $^{17}O$ concentration with administration of the contrast agent. The above method of examination can also be represented as a method for detecting cartilage damage or a method for evaluating the possibility of cartilage damage. The present invention also provides a method for collecting data for diagnosis of cartilage damage, the method including acquiring a signal value derived from $^{17}O$ from MRI images of cartilage of a subject to which an MRI contrast agent including $^{17}O$-labeled water has been administered before and after administration of the contrast agent.

The present invention also provides a method for diagnosing cartilage damage, the method including administering an MRI contrast agent including $^{17}$O-labeled water to a subject, acquiring MRI images of the subject's cartilage before and after administration of the contrast agent, acquiring a signal value derived from $^{17}$O from the MRI images, comparing the signal values or $^{17}$O concentrations calculated from the signal values between before and after administration of the contrast agent, determining a change in the signal value or an increase in the $^{17}$O concentration with administration of the contrast agent, and diagnosing that, when it is determined that the change in the signal value or the increase in the $^{17}$O concentration is present, the cartilage has damage or is likely to have damage.

The present invention further provides a program for examining cartilage damage for causing a computer to execute processing to acquire a signal value derived from $^{17}$O from MRI images of cartilage of a subject to which an MRI contrast agent including $^{17}$O-labeled water has been administered before and after administration of the contrast agent, processing to optionally calculate a $^{17}$O concentration from the signal value, and processing to compare the signal values or the $^{17}$O concentrations calculated from the signal values between before and after administration of the contrast agent. This program can be used to perform the method of examination described above, and the details of each processing are as described in the description of the method of examination. A computer-readable storage medium storing therein the program is also provided by the present invention. The recording medium may be a portable medium such as a compact disc (CD), a digital versatile disc (DVD), or a USB flash drive or a storage device such as a hard disk incorporated in a computer. The program recorded on the recording medium may be for implementing all the functions described above or for implementing part of them. In the latter case, the functions described above can be implemented by a combination with a program already recorded on the computer.

The present invention further provides a system for examining cartilage damage, the system including means for acquiring a signal value derived from $^{17}$O from MRI images of cartilage of a subject to which an MRI contrast agent including $^{17}$O-labeled water has been administered before and after administration of the contrast agent, means for optionally calculating a $^{17}$O concentration from the signal value, and means for comparing the signal values or the $^{17}$O concentrations calculated from the signal values between before and after administration of the contrast agent.

The present invention further provides an image analysis apparatus including a signal value acquisition unit acquiring a signal value derived from $^{17}$O from MRI images of cartilage of a subject to which an MRI contrast agent including $^{17}$O-labeled water has been administered before and after administration of the contrast agent, a $^{17}$O concentration calculation unit optionally calculating a $^{17}$O concentration from the signal value, and a comparison unit comparing the signal values or the $^{17}$O concentrations calculated from the signal values between before and after administration of the contrast agent.

The present invention further provides an MRI apparatus including a nuclear magnetic resonance imaging unit imaging cartilage of a subject to which an MRI contrast agent including $^{17}$O-labeled water has been administered, an image generating unit generating an MRI image from echo data acquired by the nuclear magnetic resonance imaging unit, a signal value acquisition unit acquiring a signal value derived from $^{17}$O from the MRI image, a $^{17}$O concentration calculation unit optionally calculating a $^{17}$O concentration from the signal value, and a comparison unit comparing the signal values or the $^{17}$O concentrations calculated from the signal values between before and after administration of the contrast agent.

The details of the program, the system, the image analysis apparatus, and the MRI apparatus are as described in the description of the method of examination described above. The program, the storage medium, the system, the image analysis apparatus, and the MRI apparatus can be used in the implementation of the method of examination and the method of diagnosis described above.

As described in examples below, after administration of $^{17}$O-labeled water, the $^{17}$O concentration promptly increases in a damaged cartilage region, whereas such a concentration increase does not occur in a normal cartilage region. Thus, according to the present invention, the contrast agent including $^{17}$O-labeled water is administered to a subject in need of a cartilage damage examination, and the change in the signal value derived from $^{17}$O in the cartilage is detected through MRI imaging, and thereby image diagnosis of cartilage damage can be performed.

The increase in the $^{17}$O concentration is thought to be due to $H_2$-$^{17}$O molecules in the contrast agent penetrating into the cartilage from damaged sites of cartilage. The penetration of water molecules is considered possible even if the degree of cartilage damage is slight, and thus the present invention can detect even the slightest damage such as degeneration of the cartilage surface layer, which is an early lesion of osteoarthritis, which has been difficult to detect.

The present invention is applied to a subject in need of a cartilage damage examination. Examples of the subject include mammals such as rodents including mice, rats, hamsters, guinea pigs, and rabbits; primates including humans, chimpanzees, and rhesus monkeys; domestic animals including pigs, cattle, goats, horses, and sheep; and pet animals including dogs and cats. The subject is preferably a human.

The subject in need of a cartilage damage examination is a subject that has or may have some damage to the cartilage. Examples of the subject include a subject that has or may have a disease or symptom with cartilage damage or caused by cartilage damage. Examples of the disease or symptom include osteoarthritis (the knee joint, the elbow joint, the ankle joint, the shoulder joint, and the like), autoimmune arthritis such as rheumatoid arthritis, intra-articular fractures, meniscus injuries, knee ligament injuries, patellar dislocation, chronic knee disorders due to sports, intervertebral disc disorders, and osteochondritis dissecans (the knee joint, the elbow joint, the ankle joint, and the like).

The following describes the present invention in more detail by the following examples, but the invention is not limited to these examples.

Examples

Examples: Evaluation of Articular Cartilage Damage in Anterior Cruciate Ligament Transection (ACLT) Model Using $^{17}$O-Labeled Water

(1) Materials and Methods

- Creation of ACLT Model

An ACLT model was created using 14-week-old male Japanese white rabbits to induce cartilage damage in the knee joint. The rabbits were anesthetized intravenously with pentobarbital (30 mg/kg) and ketamine (50 mg/kg), followed by inhalation anesthesia with isoflurane (1 to 4%) for maintenance anesthesia. The anterior surface of the knee joint on the operative side was shaved, was disinfected with povidone-iodine, and then an analgesic (buprenorphine 0.02 mg/kg) was subcutaneously administered by injection. Skin incision of about 5 cm was placed on the medial side of the patella, and the joint capsule was incised in a similar position. The patella was dislocated laterally, and the knee joint was deeply flexed to expose the anterior cruciate ligament of the femoral intercondylar part, which was separated using a No. 11 scalpel. After washing the inside of the joint with a physiological saline solution, the joint capsule and the skin were sutured using 3-0 nylon thread. Postoperatively, euthanasia was performed if wound infection was observed or in the case of an inability to stand.

It has been reported that in the ACLT model, degeneration of cartilage surface begins at 4 weeks postoperatively and occurs in the entire cartilage layer from 8 weeks postoperatively (Makoto Yoshioka et al. Characterization of a model of osteoarthritis in the rabbit knee. Osteoarthritis and Cartilage, 1996, 4 (2): 87-98), and thus five rabbits were slaughtered and euthanized with pentobarbital (200 mg/kg) each at 4 weeks and 8 weeks postoperatively, and cartilage damage was evaluated by MRI and macroscopically and histologically.

- MRI imaging

Using a 3T-MRI apparatus (MAGNETOM Prisma, Siemens), imaging of the rabbit knee joint on the operative side was performed. The affected rabbit limb was disarticulated at the thigh and was fixed at the knee joint extension to avoid the influence of body movement during imaging. A 24G Surflo needle was inserted from the anterior surface of the knee joint through the patellar tendon and was indwelled in the joint. By cutting the locking connector side of an extension tube and coupling the lumen of the cut surface and a narrow polyethylene tube (PE50) to each other with curable resin (Shofu Quick Resin), a self-made administration route with a narrow lumen was created and was connected to the indwelled Surflo needle.

To evaluate both femoral condyles, five slices were set as MRI imaging cross sections in such a manner that the most distal part of both femoral condyles was the central slice in a coronal section anteverted 45 degrees to the bone axis (indicated by the five dotted lines in FIG. 2A). First, a proton density-weighted image (PDWI) was taken as a reference image for identifying the articular cartilage under the following conditions: TE, 21 ms; TR, 1,600; FOV, 60 × 80 mm; matrix, 269 × 448; slice thickness, 2 mm; slice gap, 2.6 mm; number of excitations, 6; and scan time, 3 min 39 s. Subsequently, T2-weighted images using the 2D-fast spin echo method were successively taken under the following imaging conditions: TE, 129 ms; TR, 1,600; echo train length, 12; FOV, 60 × 80 mm; matrix, 269 × 448; slice thickness, 2 mm; slice gap, 2.6 mm; number of excitations, 6; scan time, 3 min 39 s; number of repetitions, 18; and total scan time, 65 min 42 s. The first three images out of 18 images taken consecutively were used as baseline images before administration. Immediately after the third imaging ended, 0.5 ml of a physiological saline solution containing a 20-mol% $^{17}$O-labeled water (Taiyo Nippon Sanso Corporation) was rapidly administered into the knee joint through the indwelled Surflo and was flushed with 0.5 ml of a physiological saline solution.

- MRI Image Evaluation

The articular cartilage in each slice section was identified from the proton density-weighted image, and a plurality of small regions of interest (ROIs) were set in the articular cartilage of both condyles. The $^{17}$O concentration at each point in time in each set ROI was calculated from the following equation using the signal value of the T2-weighted image, and changes over time in the $^{17}$O concentration in each ROI were compared with the histological grade of cartilage damage described below.

[Mathematical 2]

$$^{17}\text{O concentration (mol\%) in each ROI} = -1/(TE \times R_2^{17}O) \times \log(S/S0) + 0.038$$

In the equation, TE is a time of echo, $R_2^{17}O$ is 3.33, S is the signal value in the ROI at a certain point in time after administration of $^{17}O$-labeled water, and S0 is an average signal value in the ROI before administration of $^{17}O$-labeled water (the average of the signal values in the ROI in the three images taken as the baseline images before administration). The value 0.038 corresponds to the natural abundance of $^{17}O$.

- Macroscopic and Histological Evaluation

After MRI, the rabbit knee joint was incised and expanded, and cartilage damage in the femoral condyle was macroscopically and histologically evaluated according to the MRI slice (Gabriel GN et al. Evaluation of multiphase implants for repair of focal osteochondral defects in goats. Biomaterials, 2000, 21 (24): 2561-74).

In the macroscopic evaluation, cartilage damage was evaluated using Osteoarthritis Research Society International (OARSI) scoring (S. Laverty et al. The OARSI histopathology initiative - recommendations for histological assessments of osteoarthritis in the rabbit. Osteoarthritis and Cartilage, 2010, 18 Suppl 3: S53-S65) with Indian ink. An OARSI score 2 or more, which shows accumulation of Indian ink on the joint surface, was defined as "macroscopic cartilage damage being present," while an OARSI score 1, which shows no accumulation, was defined as "macroscopic cartilage damage being absent."

In the histological evaluation, both femoral condyles were first fixed in 10% formalin for 24 hours and were then decalcified in formic acid with a ratio of formic acid 5 : formalin stock solution 1 : distilled water 15 for 20 days. Specimens were prepared and evaluated for three slices including, as a central slice, the most distal part of both femoral condyles in a coronal section anteverted 45 degrees to the bone axis so as to match the slice width of MRI and slices 2.6 mm anterior and posterior to the central slice. Safranin-O staining was performed, and the degree of cartilage damage was evaluated by OARSI grading (H.J. Mankin et al. Biochemical and metabolic abnormalities in articular cartilage from osteoarthritic human hips. II. Correlation of morphology with biochemical and metabolic data. J Bone Joint Surg, 1971, 53 (3): 523-37). An OARSI grade 1 or more, which shows fibrosis of the cartilage surface layer, was defined as "histological cartilage damage being present," while an OARSI grade 0, which shows normal cartilage, was defined as "histological cartilage damage being absent." Scoring was performed twice independently by two examiners to evaluate inter-examiner errors and intra-examiner errors.

- Research Approval

All animal experiments were performed according to a protocol approved by the Institutional Animal Care and Use Committee of the Graduate School of Medicine, Hokkaido University.

(2) Results

For a representative case at 4 weeks postoperatively, FIG. 1A is an appearance photograph after India ink staining. The macroscopic evaluation allowed observation of cartilage damage with an OARSI score 3 on the lateral side of the medial condyle and cartilage damage with an OARSI score 2 on the medial side of the lateral condyle. FIG. 1B and FIG. 1C illustrate an HE-stained image and a Safranin-O-stained image of the sliced specimen, respectively. The histological evaluation allowed observation of cartilage damage with an OARSI grade 3 (cracks from the cartilage surface layer to the intermediate layer are present, the black arrows in the drawings) on the lateral side of the medial condyle and cartilage damage with an OARSI grade 2 (cracks in the cartilage intermediate layer, the white arrows in the drawings) on the medial side of the lateral condyle in both the HE-stained image and the Safranin-O-stained image, which were consistent with the damage parts observed in the macroscopic evaluation.

For a representative case at 4 weeks postoperatively, FIG. 2B illustrates a T2-weighted image of both femoral condyles in a coronal section at the central dotted line out of the five dotted lines illustrated in FIG. 2A, and FIG. 2C illustrates a $^{17}O$ map obtained by calculating $^{17}O$ concentrations from signal values of the respective pixels of a T2-weighted image taken about 11 minutes after administration of $^{17}O$-labeled water (the sixth imaging) and mapping them, superimposed on a proton density-weighted image. It was confirmed that the $^{17}O$ concentration was higher in the region where cartilage damage was observed.

Two ROIs were set in each of a region corresponding to the damaged cartilage in the T2-weighted image and a region corresponding to the normal cartilage, and the $^{17}O$ concentration was calculated based on the average of signal values in each ROI. FIG. 3A illustrates the set positions of the ROIs, and FIG. 3B illustrates changes over time in the $^{17}O$ concentration in the respective ROIs. ROI 1 and ROI 2, which were set in the damaged cartilage region, showed an increase in the $^{17}O$ concentration immediately after administration of $^{17}O$-labeled water (the fourth imaging), whereas ROI 3 and ROI 4, which were set in the normal cartilage region, showed no tendency of an increase in the $^{17}O$ concentration with administration of $^{17}O$-labeled water.

**Claims**

1. A magnetic resonance imaging (MRI) contrast agent for detecting cartilage damage, the MRI contrast agent comprising $^{17}O$-labeled water.

2. A method for examining cartilage damage, the method comprising:

   acquiring a signal value derived from $^{17}O$ from magnetic resonance imaging (MRI) images of cartilage of a subject to which an MRI contrast agent including $^{17}O$-labeled water has been administered before and after administration of the contrast agent;
   comparing the signal values or $^{17}O$ concentrations calculated from the signal values between before and after administration of the contrast agent; and
   determining a change in the signal value or an increase in the $^{17}O$ concentration with administration of the contrast agent.

3. The method according to claim 2, wherein the cartilage is articular cartilage.

4. The method according to claim 2 or 3, wherein the contrast agent is administered into a joint cavity of the subject.

5. The method according to any one of claims 2 to 4, wherein

   the MRI images are T2-weighted images taken with a proton MRI apparatus, and
   the change in the signal value is a reduction in the signal value.

6. A program for examining cartilage damage for executing:

   processing to acquire a signal value derived from $^{17}O$ from magnetic resonance imaging (MRI) images of cartilage of a subject to which an MRI contrast agent including $^{17}O$-labeled water has been administered before and after administration of the contrast agent;
   processing to optionally calculate a $^{17}O$ concentration from the signal value; and
   processing to compare the signal values or the $^{17}O$ concentrations calculated from the signal values between before and after administration of the contrast agent.

7. The program according to claim 6, wherein the cartilage is articular cartilage.

8. The program according to claim 6 or 7, wherein the contrast agent is administered into a joint cavity of the subject.

9. The program according to any one of claims 6 to 8, wherein

   the MRI images are T2-weighted images taken with a proton MRI apparatus, and
   the change in the signal value is a reduction in the signal value.

Fig.1

A.

B.

C.

Fig.2

A.

B. T2WI

C. 17O concentration map of articular cartilage (fused with PDWI)

Fig.3

A.

B.

## INTERNATIONAL SEARCH REPORT

International application No.

**PCT/JP2021/039798**

### A. CLASSIFICATION OF SUBJECT MATTER

*A61K 49/06*(2006.01)i; *A61B 5/055*(2006.01)i
FI: A61K49/06; A61B5/055 383

According to International Patent Classification (IPC) or to both national classification and IPC

### B. FIELDS SEARCHED

Minimum documentation searched (classification system followed by classification symbols)

A61K49/06; A61B5/055

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

JSTPlus/JMEDPlus/JST7580 (JDreamIII); CAplus/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| Y | JP 2008-239566 A (TAIYO NIPPON SANSO CORP.) 09 October 2008 (2008-10-09) claims, paragraphs [0003], [0012]-[0020], examples, etc. | 1-9 |
| Y | HAGIWARA, K. et al. Immunolocalization of water channel aquaporins in human knee articular cartilage with intact and early degenerative regions. Med Mol Morphol. 2013, vol. 46, pages 104-108, ISSN 1860-1480 Abstract, Discussion, etc. | 1-9 |
| Y | 羽田勝彦, 外8名, OA軟骨における, アクアポリン1の発現および機能解析, 日整会誌, 2017, vol. 91, no. 8, p. S1616, ISSN 0021-5325, (The Journal of the Japanese Orthopaedic Association.), non-official translation (HANEDA, Masahiko et al. Expression and functional analysis of aquaporin 1 in OA cartilage.) column 1-PD-3 | 1-9 |
| Y | CAI, L. et al. Overexpression of aquaporin 4 in articular chondrocytes exacerbates the severity of adjuvant-induced arthritis in rats: an in vivo and in vitro study. J Inflamm. 2017, vol. 14, Article No. 6, pages 1-10, ISSN 1476-9255 Abstract, Background, Discussion, Conclusions, etc. | 1-9 |

☐ Further documents are listed in the continuation of Box C.   ☑ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **21 December 2021** | **11 January 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

INTERNATIONAL SEARCH REPORT
Information on patent family members

International application No.

**PCT/JP2021/039798**

| Patent document cited in search report | | | Publication date (day/month/year) | Patent family member(s) | Publication date (day/month/year) |
|---|---|---|---|---|---|
| JP | 2008-239566 | A | 09 October 2008 | US       2010/0062478       A1<br>claims, paragraphs [0003]-[000<br>7], [0040]-[0059], examples<br>WO       2008/117850       A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2003102698 A
- JP 2013255586 A
- JP 2000218134 A

### Non-patent literature cited in the description

- **KUDO K. et al.** *Magn Reson Med Sci,* 2018, vol. 17 (3), 223-230
- **MAKOTO YOSHIOKA et al.** Characterization of a model of osteoarthritis in the rabbit knee. *Osteoarthritis and Cartilage,* 1996, vol. 4 (2), 87-98
- **GABRIEL GN et al.** Evaluation of multiphase implants for repair of focal osteochondral defects in goats. *Biomaterials,* 2000, vol. 21 (24), 2561-74
- **S. LAVERTY et al.** The OARSI histopathology initiative - recommendations for histological assessments of osteoarthritis in the rabbit. *Osteoarthritis and Cartilage,* 2010, vol. 18 (3), S53-S65
- **H.J. MANKIN et al.** Biochemical and metabolic abnormalities in articular cartilage from osteoarthritic human hips. II. Correlation of morphology with biochemical and metabolic data. *J Bone Joint Surg,* 1971, vol. 53 (3), 523-37